## Europäisches Patentamt
## European Patent Office
## Office européen des brevets

(11) Publication number: **0 066 961**
**B1**

(12) **EUROPEAN PATENT SPECIFICATION**

(45) Date of publication of patent specification: **26.11.86**

(51) Int. Cl.⁴: $C\ 12\ P\ 19/06$

(21) Application number: **82302416.1**

(22) Date of filing: **12.05.82**

(54) Production of xanthan having a low pyruvate content.

(30) Priority: **22.05.81 GB 8115854**

(43) Date of publication of application:
**15.12.82 Bulletin 82/50**

(45) Publication of the grant of the patent:
**26.11.86 Bulletin 86/48**

(84) Designated Contracting States:
**DE FR GB**

(56) References cited:
**EP-A-0 028 446**
**FR-A-2 408 653**
**FR-A-2 442 888**
**US-A-3 455 786**

**CARBOHYDRATE RESEARCH, vol. 63, 1978, pages 253-256, Elsevier, Amsterdam, NL. P.A. SANDFORD et al.: "Separation of xanthan gums of differing pyruvate content by fractional precipitation with alcohol"**

(73) Proprietor: **Kelco Biospecialties Limited**
**22 Henrietta Street**
**London WC2E 8NB (GB)**

(72) Inventor: **Jarman, Trevor Rodney**
**18, Moss Drive**
**Haslingfield Cambridgeshire (GB)**
Inventor: **Pace, Gary William**
**65, Bannockburn Road**
**Pymble, NSW 2073 (AU)**

(74) Representative: **Ablewhite, Alan James et al**
**MARKS & CLERK 57/60 Lincoln's Inn Fields**
**London WC2A 3LS (GB)**

(56) References cited:
**CHEMICAL ABSTRACTS, vol. 89, no. 17, 23rd October 1978, page 460, no. 144991j, Columbus, Ohio, USA I.W. DAVIDSON: "Production of polysaccharide by xanthomonas campestris in continuous culture"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the production of xanthan, the biopolymer obtained from various species of the genus *Xanthomonas,* having a low pyruvate content.

The structure of xanthan, specifically the gum produced by *Xanthomonas campestris* strains, was proposed by Jansson *et al* (Carbohydrate Research 45, 275—282 (1975)) to consist of a β (1—4)-linked glucose backbone, alternative residues of which are substituted with a trisaccharide side chain composed of mannose and glucuronic acid in the ratio 2:1. These side chains terminate in D-mannose residues, a proportion of which are substituted with pyruvic acid to form 4,6-*O*-(1-carboxyethylidene)-D-mannose. Some other *Xanthomanas* species have been shown to produce exopolysaccharides composed of glucose, mannose and glucuronic acid containing substituent pyruvic acid at a level greater than 1% by weight (Orentas *et al*, Can. J. Microbiol. *9*,427,430 (1963)). It is recognized that the proportion of the D-mannosyl residue substituted with pyruvic acid in xanthan generally can vary and it is usual for the level to be in the range 2.5—5.0% by weight. In a comprehensive study, Cadmus *et al* (Biotecnol, Bioeng. *20*, 1003—1014, 1978) found that the culture medium composition, the strain of *X.campestris* and the cultivation temperature all influence the pyruvic acid content of the polymer. A pyruvic acid content of 1.3% was the minimum obtained.

U.K. Patent Application No. GB 2 008 600 A, in the name of Pfizer Inc., describes the production of xanthan gums which are pyruvate-free. These gums are produced by a new strain of *Xanthomonas,* namely *Xanthomonas campestris* ATCC 31313.

One use for which xanthan is of considerable interest, is as a water additive for use in the displacement of oil from partially depleted oil fields. In this technique, oil which is still in the pores of the oil-bearing rock after the initial pressurized oil has been recovered, is displaced by flooding the rock formation with water. This water is rendered more viscous by the addition of a polymeric water-soluble material such as a polyacrylamide or xanthan in order to improve its displacement characteristics. According to GB 2 008 600A, completely pyruvate-free xanthan is advantageous in water flooding processes since it is less prone to precipitation causing blockage of the pores of the oil-bearing rock. However the pyruvate-free xanthan of GB 2 008 600A exhibits a sharp decrease in apparent viscosity at 70°C.

As explained above, ordinary strains of *Xanthomonas* produce, under the usual cultivation condition, a xanthan having a pyruvate content of over 1%. Indeed, for food use, xanthan is required to have a pyruvate content of at least 1.5%, and thus industrial xanthan-producing processes concentrate on producing xanthan having a relatively high pyruvate content.

In FEMS Microbiology Letters (1978) Vol. 3 pp 347—349 is described as a xanthan gum with a pyruvate content of 0.9%, produced by cultivation of *X.campestris* in a medium containing trace elements, under conditions of phosphate and magnesium limitation.

We have found that by cultivating *X.campestris* under other conditions, preferably in continuous culture, a xanthan gum can be produced having a pyruvate content of below 1%. In some instances a pyruvate content of about 0.1%, i.e. near 0, can be obtained by this method. We have found that the control of the pyruvate content is related to a number of factors, all of which must be right for low pyruvate product to be obtained.

The pyruvate level referred to generally throughout this specification is measured by F.C.C. Food Chemicals Codex, Second Edition, National Academy of Sciences, Washington DC 1971, page 856 method which is the method used in nearly all the existing xanthan art up to now. Typically, a broth or xanthan solution containing 0.2—0.4% w/v polysaccharide is hydrolysed in 1 N HCl for 3 hours. A 2 ml aliquot is removed and is mixed with 1 ml of a 2,4-dinitrophenylhydrazine reagent (0.5% w/v in 2N HCl) for 5 minutes. The reaction mixture is extracted with 5 ml of ethyl acetate and the aqueous layer is discarded. The ethyl acetate extract is extracted with three 5 ml portions of 10% w/v aqueous sodium carbonate and then the combined extracts are diluted to 25 ml with additional 10% w/v aqueous sodium carbonate. The optical density of this solution is then measured at 375 nm and the pyruvate content determined against a previously known standard. Using this method, the product of the process of the present invention has a pyruvate level of less than 1%, typically from 0.25 to 0.65%. However, there is an alternative assay for pyruvate, described in U.S.D.A. ARS—NC—51 November 1976. This assay is believed to be more reliable and more accurate, but gives lower figures than the FCC test. Typically, the product of the present process is found to contain from 0.07 to about 0.25% pyruvate by this enzymic assay.

According to the present invention we provide a process for preparing low pyruvate xanthan gum which comprises aerobically fermenting an organism of the genus *Xanthomonas* in a broth comprising:

1) a chemically defined simple salts medium comprising sources of nitrogen, sulphur, potassium, phosphorus, magnesium, calcium, and (optionally) iron, said simple salt medium being substantially free of cobalt, copper, manganese, zinc and boron;

2) an assimilable carbon source, and

3) a complex organic material which is a nitrogen source. The process is preferably continuous, with a conversion efficiency of greater than 44% and a xanthan to cell ratio by weight of at least 4:1.

The invention also provides as a novel product low pyruvate xanthan gum which comprises 0.25 to 0.65% w/w pyruvate (FCC method), or 0.07 to 0.25% pyruvate (USDA ARS—NC—51 method).

As stated, the medium used for the continuous fermentation should be defined, simple salts, medium, that is to say a medium containing only inorganic salts of known formula as sources of N, P, K, Ca, Fe and Mg and a carbohydrate of known formula as the carbon source. In this basic medium is included a complex organic material which may act as nitrogen source. The complex nitrogen source may comprise, for example, a yeast extract or a leguminous product such as soy flour. Yest extract is preferred. The complex nitrogen source is to be added, at a level of 0.1 to 0.5 g available N per litre in the culture medium preferably about 0.02 g/l. With a low-salts yeast extract, 0.02 g available N per litre corresponds to a level of about 0.2 g yeast extract per litre. The complex nitrogen source is an essential feature of the present invention, together with the absence of trace elements from the medium. If a normal, simple salts, chemically defined medium is modified to contain no trace elements, we find that the cell growth is considerably suppressed and the polysaccharide produced has a rather low pyruvate content. The use of such a medium is obviously not commercially useful, as the yield of xanthan would be too low. However, we have found that if trace elements (with the possible exception of iron) are excluded but the medium is enriched with a complex source such as yeast extract, the product can be very low pyruvate material and can be produced in good yield. Thus, for example, Low Salt Yeast Extract, sold by Distillers Company Limited, contains Zn 60 p.p.m.; Cu 10 p.p.m.; Mn 15 p.p.m.; CO 5 p.p.m.; Fe 100 p.p.m. At a concentration of 200 p.p.m. in the medium, the yeast extract thus supplies no more than 0.012 p.p.m. of Zn, Mn, Co and Cu and only 0.02 p.p.m. of Fe. In contrast a normal medium might contain from 0.05 to 0.5 p.p.m. of Zn, Mn, Co and Cu and from 5 to 10 p.p.m. of Fe.

The carbohydrate source is conveniently a monosaccharide which may be in the form of a syrup which may also contain higher saccharides, for example a glucose syrup derived from acid and/or enzymic degradation of starch.

All of these requirements are essential and if departed from in any way tend to lead to the production of xanthan containing higher pyruvate content than required, unless a special strain of X.campestris, such as ATCC 31313, is used.

Using the conditions according to the invention, a low-pyruvate xanthan is obtained, possessing viscosity characteristics which make it useful in the same applications as that obtained from X.campestris ATCC 31313 by the process of Application 2 008 600A.

The low-pyruvate xanthan obtained according to the present invention can be isolated from the culture medium in any conventional manner. Usually, the polysaccharide will be precipitated by addition of an organic solvent such as isopropanol. If desired, the polysacccharide can then be further purified and clarified, e.g. by treatment with acid or alkali and/or with an enzyme.

The requirement that the T.P.M. concentration should be at least 15 $gl^{-1}$ with a polymer to cell ratio of at least 4:1 can be achieved by methods well known in this art, namely by ensuring that the nutrient concentration is sufficiently high (although the exact level will depend on the type of nutrient used). As a typical example, in a nitrogen-limited fermentation, a nitrogen level of at least 0.2 g is required. Preferably, a T.P.M. level is achieved which gives an actual xanthan level of at least 15 $gl^{-1}$.

The rate of continuous fermentation will, under steady state conditions, inevitably be limited by the concentration of one of the nutrients in the medium. This limiting nutrient may, for example, be nitrogen or sulphur, or may be one of the trace elements missing from the medium. A preferred medium contains, for example, from 40—70 g/l of glucose (as a syrup); 2 to 4 g/l of an ammonium salt, e.g., ammonium sulphate or diammonium hydrogen phosphate; about 0.02 to 0.1 g/l of magnesium compounds; about 0.25 to 0.75 g/l of potassium salts, up to 0.05 g/l of iron salts and 0.1 to 0.5 g/l low-salts yeast extract; in deionised water.

The oxygenation should also be sufficiently plentiful e.g. by providing good aeration coupled with efficient stirring of the fermenter tank.

The following examples illustrate the invention further.

Example 1

*Xanthomonas campestris* (laboratory stock derived from ATCC 13951) was grown continuously in a 5 litre capacity, stirred tank fermenter. Fermentation parameters were:— pH, 7.0; temperature, 30°C, impeller speed, 1000 $rev.min^{-1}$; impeller diameter, 8.4 cm (curved-bladed impellers with blades swept from direction of rotation); air flow, 1.47 l. $min^{-1}$. The composition of the culture medium is given in Table 1. The glucose (in one-fifth total volume) was sterilised separately from the salts components (in four-fifths total volume) at 121°C and a pressure of 1 bar for 15 mins and when cooled the solution were mixed to give the complete medium at pH 6.8. The working volume was set at 2 litres by means of a weir. Stirring was achieved by using two curved-bladed impellers 6.0 cm apart. The lowest impeller was positioned at the lowest point on the impeller shaft. Air was introduced below the lowest impeller. Mixing was aided by four baffles, positioned longitudinally, around the circumference of the culture vessel. The fermenter was fitted with automatic pH control for metering in 1M NaOH to maintain the pH at 7.0. The fermenter and addition lines were sterilised in the usual way prior to addition of the medium.

To the medium in the culture vessel was added 100 ml of a culture grown in MYGP broth (g/l:— malt extract, 3.0; yeast extract, 3.0; glucose, 10.0; peptone, 5.0; pH not adjusted) in shake flasks. The culture was grown in batch mode for 36h under the described conditions. A continuous medium feed was then supplied at a dilution rate of $0.05h^{-1}$ (ml/h). The culture was monitored for (a) pH; (b) measurement of $O_2$ uptake and $CO_2$ evolution using an oxygen analyser and an infrared carbon dioxide analyser; (c) total isopropanol precipitable matter (TPM)- (precipitate from broth: isopropanol mixture (1:2 w/v) collected by filtration and dried under an infra-red lamp); (d) optical measurement of cell density at 540 nm.

The culture was allowed to reach a "steady-state". A sample of the total precipitable matter (xanthan gum) was analysed for pyruvate content by the enzymic method described in the U.S. Department of Agriculture publication ARS—NC—51, November 1976. The viscosities of a 1% solution of the xanthan gum is distilled water and in a 1% solution of sodium chloride were measured on a Contraves Rheomat 30 cone and plate viscometer.

A xanthan gum with a pyruvate content of 0.6% (FCC) was obtained as indicated in Table 2.

### Example 2

*Xanthomonas campestris* (strain of Example 1) was continuously cultured under the conditions described in Example 1 except that the air flow rate was 2.0 litres $min^{-1}$, and the impeller speed was 550 rev.$min^{-1}$, the two coaxial impellers being 13 cm diameter and 10 cm apart, each having 6 curved blades. The culture medium used, which was sulphur limited with nitrogen supplied as ammonium, is given in Table 1. The characteristics of the product obtained as summarised in Table 2.

### Example 3

*Xanthomonas campestris* (strain of Example 1) was continuously cultured as described in Example 2 except that working volume was 2.5 l, air flow rate was 4.0 l $min^{-1}$, impeller speed was 650 rev. $min^{-1}$ and dilution rate was $0.046h^{-1}$. The medium used was designed to be nitrogen limited (Table 1), but a small amount of nitrogen was detected in the broth removed, indicating that the limiting factor was possibly a trace element rather than nitrogen. Steady state conditions were achieved for about 230 hours. The pyruvate content of xanthan gum obtained under these steady-state conditions was $0.09 \pm 0.02\%$ (w/w) (USDA ARS—NC—51) or 0.3% (w/w) (FCC).

### Example 4

*Xanthomonas campestris* (strain of Example 1) was continuously cultured as described in Example 1. Air flow rate was 1.75 l $min^{-1}$, impeller speed was 1200 rev. $min^{-1}$, dilution rate was $0.055$ $h^{-1}$. The culture medium (Table 1) was similar to that of Example 3. The pyruvate content of xanthan gum obtained under steady-state conditions was 0.3% (w/w) (FCC).

### Comparative Examples 1—4

*Xanthomonas campestris* (strain of Example 1) was continuously cultured in a similar way to that described in Example 1. The culture medium, as given in Table 1, contained no trace elements nor yeast extract (comp. Example 1); trace elements but no yeast extract (Comp. Examples 2 and 4); or trace elements and yeast extract (Comp. Example 3).

In Comp. Example 2, the trace element level was reduced 50% compared with that of Comp. Example 4. The results are summarised in Table 2. Comparison of product obtained according to the invention with a higher pyruvate product shows that both possess similar viscosities.

A striking feature of the low pyruvate material prepared according to the present invention, however, is its apparent stability to elevated temperatures. A solution can be refluxed for several hours without the viscosity being markedly lowered. This property is significant when it is rememberd that the temperatures encountered in oil-bearing reservoirs can often be greater than 100°C. Figure 1 shows a comparison of viscosities after various reflux times for the product of Example 4, the product of Comparative Example 4 and two commercially available xanthan guns, Rhodopol (T.M) (Rhone Poulenc) and Keltrol (T.M) (Kelco Div. of Merck & Co., Inc.) In order to show that the stability is not merely due to the absence of trace elements in the solution, a curve for a solution containing the product plus trace elements equivalent to normal medium if all entered into product is included. Heat treatment of the product in curve (5) was at 125°C for 2 minutes.

TABLE 1

Culture media used in the Examples

| Component | Example | | | | Comparative Example | | | |
|---|---|---|---|---|---|---|---|---|
| $gl^{-1}$ | 1 | 2 | 3 | 4 | 1 | 2 | 3 | 4 |
| Glucose | $43^1$ | $43^1$ | $56^1$ | $67^1$ | $62^2$ | $62^1$ | $60^1$ | $62^1$ |
| Acetic acid | 0 | 0 | 0 | 0 | 0 | 0.45 | 0.45 | 0.45 |
| Citric acid | 1.0 | 1.0 | 0.6 | 0.6 | 1.0 | 1.0 | 1.0 | 1.0 |
| Mg $(OH)_2$ | 0.034 | 0.034 | 0 | 0 | 0.048 | 0.078 | 0.07 | 0.078 |
| Ca $(OH)_2$ | 0.025 | 0.025 | 0.025 | 0.025 | 0 | 0.05 | 0.05 | 0.05 |
| $MgSO_4.7H_2O$ | 0.062 | 0.062 | 0.02 | 0.02 | 0 | 0.06 | 0.09 | 0.06 |
| $K_2HPO_4$ | 0 | 0.5 | 0 | 0 | 0.78 | 1.0 | 1.0 | 1.0 |
| $KH_2PO_4$ | 0.5 | 0 | 0.5 | 0.5 | 0 | 0 | 0 | 0 |
| $(NH_4)_2HPO_4$ | 3.77 | 3.77 | 2.7 | 2.7 | 3.02 | 2.62 | 2.38 | 3.0 |
| Yeast Extract | 0.2 | 0.2 | 0.2 | 0.2 | 0 | 0 | 0.2 | 0 |
| $Mgl^{-1}$ (p.p.m) | | | | | | | | |
| $MnSO_4.4H_2O$ | 0 | 0 | 0 | 0 | 0 | 0.55 | 1.1 | 1.1 |
| $ZnSO_4.7H_2O$ | 0. | 0 | 0 | 0 | 0 | 0.71 | 0.71 | 1.42 |
| $CuSO_4.5H_2O$ | 0 | 0 | 0 | 0 | 0 | 0.125 | 0.25 | 0.25 |
| $CoSO_4.7H_2O$ | 0 | 0 | 0 | 0 | 0 | 0.14 | 0.28 | 0.28 |
| $H_3BO_4$ | 0 | 0 | 0 | 0 | 0.553 | 0.03 | 0.06 | 0.06 |
| $FeSO_4.7H_2O$ | 35 | 0 | 36 | 36 | 0.014 | 17.8 | 3.56 | 35.6 |

1 glucose = Dexyme 081® from Tunnel Refineries Limited, Tunnel Avenue, Greenwich, London SE10
2 glucose = Meritose® Grade AR, from Fisons Scientific Apparatus Ltd, Meadow Road, Loughborough, Leicestershire

TABLE 2

| | Example | | | | Comparative Example | | | |
|---|---|---|---|---|---|---|---|---|
| | 1 | 2 | 3 | 4 | 1 | 2 | 3 | 4 |
| Dilution rate, h$^{-1}$ | 0.053 | 0.052 | 0.051 | 0.05 | 0.043 | 0.042 | 0.044 | 0.04 |
| Cell concentration E549 units | 5.2 | 5.2 | 5.36 | 5.6 | 3.88 | 4.6 | 5.34 | 3.8 |
| TPM, g l$^{-1}$ | 24.4 | 22.6 | 32.21 | 30 | 19.70 | 26.8 | 27.6 | 24.02 |
| Cell dry weight | 4.82 | 3.4 | 5.37 | 4.95 | 2.25 | 3.0 | 3.5 | 2.6 |
| Polymer: cell ratio | 4:1 | 5.65:1 | 5:1 | 5:1 | 7.76:1 | 7.6:1 | 6.9:1 | 8.2 |
| Broth rheology (K(n)), cp | 42099 | 431330(0.13) | 41310±5393 | 51200 | 24139(0.14) | 35124(0.2) | 28796(0.22) | 34526(0.17) |
| Oxygen uptake, mmol l$^{-1}$h$^{-1}$ | 21.0 | 22.2 | 20.2 | 24.3 | 16.75 | 10.7 | 15.84 | 18.4 |
| Pyruvate, % (FCC) | 0.6 | 0.6 | 0.3 | 0.3 | 3.4 | 7.0 | 5.4 | 6.2 |
| Product Data* | | | | | | | | |
| K and (n) ⎫ 1% | 9691(0.17) | 17102(.09) | 13234(0.08) | 12077(0.19) | 8567(0.18) | 11204(0.14) | 10450(0.14) | 11208(0.15) |
| at stated solution ⎬ 0.3% | 1320(0.26) | 1384(0.24) | 736(0.35) | 646(0.37) | 459(0.41) | 621(0.35) | 691(0.35) | 692(0.36) |
| percentage ⎭ 0.1% | 63(0.55) | 100(0.5) | 67.9(0.56) | 68 | 43(0.60) | 46(0.60) | 63(0.55) | 62(0.55) |

*K = consistency index (in m Pasn); n = flow behaviour index.

## Claims

1. Low pyruvate xanthan gum which comprises 0.25 to 0.65% w/w pyruvate (FCC method), or 0.07 to 0.25% pyruvate (USDA ARS—NC—51 method).

2. A xanthan gum of claim 1 which comprises 0.3 to 0.6% by weight pyruvate (FCC method).

3. A xanthan gum of claim 1 which comprises 0.09 ± 0.02% by weight pyruvate (USDA ARS—NC—51 method) or 0.3% by weight pyruvate (FCC method).

4. A process for preparing low pyruvate xanthan gum which comprises aerobically fermenting an organism of the genus *Xanthomonas* in broth comprising:

1) a chemically defined simple salts medium comprising sources of nitrogen, sulphur, potassium, phosphorus, magnesium, calcium and (optionally) iron, said simple salts medium being substantially free of cobalt, copper, manganese, zinc and boron;

2) an assimilable carbon source, and

3) a complex organic material which is a nitrogen source.

5. A process of claim 4 which is a continuous process, has a conversion efficiency of greater than 44%, and produces a broth with a xanthan gum: cell ratio by weight of at least 4:1.

6. A process of claim 5 wherein the complex organic material is a yeast extract or a leguminous product.

7. A process of claim 6 wherein the complex organic material supplies no more than 0.012 ppm Zn, Mn, Co, and Cu and no more than 0.2 ppm Fe to the medium.

8. A process of claim 4 wherein the organism is *X.campestris.*

9. A process of claim 8 wherein the organism is *X.Campestris* ATCC 13951.

10. A process of claim 4 further comprising isolation of the low pyruvate xanthan gum followed by purification and clarification of said gum by treatment with acid or alkali and/or with an enzyme.

11. A process for the recovery of crude oil from an oil-bearing subterranean formation comprising injecting into said formation through one or more injection wells with a mobility control solution comprising an effective amount of low pyruvate xanthan gum according to claim 1.

## Patentansprüche

1. Xanthangummi mit niedrigem Pyruvatgehalt, welcher 0,25 bis 0,65% G/G Pyruvat (FCC-Verfahren) oder 0,07 bis 0,25% Pyruvat (USDA—ARS—NC—51—Verfahren) enthält.

2. Xanthangummi nach Anspruch 1, welcer 0,3 bis 0,6 Gew.-% Pyruvat (FCC-Verfahren) enthält.

3. Xanthangummi nach Anspruch 1, welcher 0,09 ± 0,02 Gew.-% Pyruvat (USDA—ARS—NC—51-Verfahren) oder 0,3 Gew.% Pyruvat (FCC-Verfahren) enthält.

4. Verfahren zur Herstellung von Xanthangummi mit niedrigem Pyruvatgehalt durch aerobische Fermentierung eines Organismus des Genus *Xanthomonas* in einer Brühe, welche enthält:

1) ein chemisch definiertes Medium einfacher, Salze, enthaltend Stickstoff-, Schwefel-, Kalium-, Phosphor-, Magnesium-, Calcium- und (gegebenenfalls) Eisenquellen, wobei dieses Medium einfacher Salze im wesentlichen frei von Kobalt, Kupfer, Mangan, Zink und Bor ist;

2) eine assimilierbare Kohlenstoffquelle, und

3) ein komplexes organisches Material, welches eine Stickstoffquelle ist.

5. Verfahren nach Anspruch 4, welches ein kontinuierliches Verfahren ist, eine Umwandlungswirksamkeit von mehr als 44% aufweist und eine Brühe mit einem Xanthangummi-Zellen-Gewichtsverhältnis von wenigstens 4:1 erzeugt.

6. Verfahren nach Anspruch 5, worin das komplexe organische Material ein Hefeextrakt oder ein leguminoses Produkt ist.

7. Verfahren nach Anspruch 6, worin das komplexe organische Material nicht mehr als 0,012 ppm Zn, Mn, Co und Cu und nicht mehr als 0,2 ppm, Fe zu dem Medium beiträgt.

8. Verfahren nach Anspruch 4, worin der Organismus *X. camprestris* ist.

9. Verfahren nach Anspruch 8, worin der Organismus *X. campestris* ATCC 13951 ist.

10. Verfahren nach Anspruch 4, welches weiterhin die Isolierung des Xanthangummis mit niedrigem Pyruvatgehalt gefolgt von der Reinigung und Klärung dieses Gummis durch Behandlung mit Säure oder Alkali und/oder mit einem Enzym umfasst.

11. Verfahren zur Gewinnung von Rohöl aus einer ölhaltigen unterirdischen Formation durch Injizieren einer eine wirksame Menge eines Xanthangummis mit niedrigem Pyruvatgehalt nach Anspruch 1 enthaltenden, mobilitätssteuernden Lösung durch ein oder mehrere Injektionsbohrungen in diese Formation.

## Revendications

1. Gomme xanthane à faible teneur en pyruvate qui contient de 0,25 à 0,65% poids/poids de pyruvate (méthode FCC), ou de 0,07 à 0,25% de pyruvate (méthode USDA ARS—NC—51).

2. Gomme xanthane selon la revendication 1 qui contient de 0,3 à 0,6% en poids de pyruvate (méthode FCC).

3. Gomme xanthane selon la revendication 1 qui contient 0,09 ± 0,02% en poids de pyruvate (méthode USDA ARS—NC—51) ou 0,3% en poids de pyruvate (méthode FCC).

4. Procédé pour préparer de la gomme xanthane à faible teneur en pyruvate.

5. Procédé selon la revendication 4 qui est un procédé en continu, a une efficacité de conversion supérieure à 44% et produit un bouillon avec une proportion en poids gomme xanthane:cellule d'au moins 4:1.

6. Procédé selon la revendication 5 dans lequel le matériau organique complexe est un extrait de levure ou un produit légumineux.

7. Procédé selon la revendication 6 dans lequel le matériau organique complexe ne fournit pas plus de 0,012 ppm de Zn, Mn, Co et Cu et pas plus de 0,2 ppm de Fe au milieu.

8. Procédé selon la revendication 4 dans lequel l'organisme est *X. campestris.*

9. Procédé selon la revendication 8 dans lequel l'organisme est *X. campestris ATCC 13951.*

10. Procédé selon la revendication 4 comprenant en outre l'isolation de la gomme xanthane à faible teneur en pyruvate suivie par la purification et la clarification de cette gomme par traitement avec un acide ou alcali et/ou avec un enzyme.

11. Procédé de récupération de pétrole brut à partir d'une formation souterraine contenant du pétrole comprenant l'injection dans cette formation par l'intermédiaire d'un ou plusieurs puits d'injection d'une solution gouvernant la mobilité comprenant une quantité efficace de gomme xanthane à faible teneur en pyruvate selon la revendication 1.